Europäisches Patentamt

(19) European Patent Office

Office européen des brevets

(11) Publication number : **0 339 276 B1**

(12) **EUROPEAN PATENT SPECIFICATION**

(45) Date of publication of patent specification :
03.06.92 Bulletin 92/23

(51) Int. Cl.⁵ : **A61K 7/50**

(21) Application number : **89105496.7**

(22) Date of filing : **29.03.89**

(54) **Bathing preparation.**

(30) Priority : **01.04.88 JP 80451/88**

(43) Date of publication of application :
**02.11.89 Bulletin 89/44**

(45) Publication of the grant of the patent :
**03.06.92 Bulletin 92/23**

(84) Designated Contracting States :
**AT CH DE ES FR GB IT LI SE**

(56) References cited :
**EP-A- 0 150 250
CHEMICAL ABSTRACTS, vol. 104, 1986, page
377, no. 74825k, Columbus, Ohio, US; & JP-
A-60 215 617 (KAO CORP.) 29-10-1985**

(73) Proprietor : **Kao Corporation
14-10, Nihonbashi Kayabacho 1-chome
Chuo-Ku Tokyo 103 (JP)**

(72) Inventor : **Ichii, Yuji
4599-1, Ichihana Ichikai-machi
Haga-gun Tochigi (JP)**
Inventor : **Yorozu, Hidenori
423-4, Hiramatsuhoncho
Utsunomiya-shi Tochigi (JP)**
Inventor : **Fukuda, Kazuyuki
3-20-1, Innai
Funabashi-shi Chiba (JP)**
Inventor : **Izumi, Yu
2-1-501, Yamate
Funabashi-shi Chiba (JP)**

(74) Representative : **Brauns, Hans-Adolf, Dr. rer.
nat. et al
Hoffmann, Eitle & Partner, Patentanwälte
Arabellastrasse 4
W-8000 München 81 (DE)**

EP 0 339 276 B1

## Description

### FIELD OF THE INVENTION

This invention relates to a bathing preparation comprising at least one organic acid, at least one carbonate, and at least one perfume, wherein, when dissolved in bath water, the vaporization of the perfume together with the expansion of carbon dioxide gas is inhibited so as to thereby sustain the aroma of the preparation for a prolonged period of time.

### BACKGROUND OF THE INVENTION

A bathing preparation is commonly prepared by blending a mixture of inorganic salts, such as mirabilite, borax, sulfur, common salt or carbonates with, for example, perfumes, colorants, vegetable extracts and organic acids. This bathing preparation imparts an aroma and/or a color to bath water and appropriately stimulates the skin surface to thereby accelerate blood circulation and promote recovery from fatigue as well as metabolism. Expandable bathing preparations comprising at least one carbonate and at least one organic acid are known which exert improved relaxant and refreshing effects through the expansion of carbon dioxide gas in bath water, to thereby make the bath joyful.

In order to enhance the above-mentioned effects, perfumes are added to the bathing preparation. However, highly volatile perfumes scarcely give the desired effects since they are vaporized in the atmosphere together with the expansion of carbon dioxide gas.

As a result, it is proposed to use perfumes in an encapsulated or included form to thereby prevent the vaporization of the same, thus sustaining the aroma for a long period of time (cf. JP-A-62-223111 (the term "JP-A" as used herein means an "unexamined published Japanese patent application") and JP-B-52-21573 (the term "JP-B" as used herein means an "examined Japanese patent publication")). However, both encapsulation and inclusion require a troublesome procedure and a high cost. In addition to these economical disadvantages, it is difficult to form tablets of a bathing preparation comprising an encapsulated perfume.

EP-A-0 150 250 describes a bathing composition comprising a carbonate, an organic acid, a stabilizer and optionally perfume. However, this patent does not particularly mention the highly volatile perfumes of the present invention. Neither does it mention the fact that fumaric acid must be present in order to prevent vaporization of these perfumes.

### SUMMARY OF THE INVENTION

It has been found in the present invention that an expandable bathing preparation comprising 20% by weight or more of fumaric acid as an organic acid can prevent the vaporization of particular highly volatile perfumes without requiring any encapsulation on inclusion procedure.

Accordingly, the present invention provide a bathing preparation comprising:

(A) at least one organic acid, wherein fumaric acid must be present in an amount of 20% by weight or more of the total of said organic acid,

(B) at least one carbonate, and

(C) at least one perfume selected from the group consisting of terpene hydrocarbons having 10 carbon atoms and formates, acetates and propionates of an alcohol having from 5 to 10 carbon atoms.

### DETAILED DESCRIPTION OF THE INVENTION

The carbonates to be used in the bathing preparation of the present invention is not critical to the present invention. Examples thereof include sodium hydrogencarbonate, sodium carbonate, potassium hydrogencarbonate, potassium carbonate, calcium carbonate, magnesium carbonate and sodium sesquicarbonate. Either one of these carbonates or a mixture thereof may be used in the present invention.

The carbonate content in the bathing preparation preferably ranges from 5 to 80% by weight, more preferably from 10 to 50% by weight, based on the total composition.

The bathing preparation of the present invention must contain fumaric acid in an amount of 20% by weight or more of the total of the organic acids. Examples of other organic acids include citric acid, succinic acid, malic acid and tartaric acid. When the fumaric acid amounts to less than 20% by weight of the organic acids, the vaporization of the highly volatile perfumes cannot be inhibited. The total organic acid content preferably ranges 10 to 300% by weight, more preferably 30 to 150% by weight, based on the above-mentioned carbonates.

Fumaric acid, which is hardly soluble in water, sometimes shows floatation when dissolved in bath water.

Therefore, it may be made highly soluble in water by surface-treating such with water-soluble polymers, e.g., sodium polyacrylate or sodium carboxymethyl cellulose, or hydrophilic nonionic surfactants, e.g., polyethylene glycol, sucrose fatty acid esters, polyglycerol fatty acid esters, or a mixture thereof. The water-soluble polymers and hydrophilic nonionic surfactants are preferably used in an amount of 0 to 40% by weight (more preferably 0.1 to 20% by weight) based on the fumaric acid and in an amount of 0 to 5% by weight (more preferably 0.02 to 0.1% by weight) based on the fumaric acid, respectively.

Regarding the highly volatile perfumes to be used in the present invention, examples of the terpene hydrocarbons having 10 carbon atoms include α-pinene, β-pinene, camphene, limonene, terpinolene, myrcene and p-cymene; examples of the formates of an alcohol having from 5 to 10 carbon atoms include geranyl formate, benzyl formate and phenylethyl formate; examples of acetates of an alcohol having from 5 to 10 carbon atoms include isoamyl acetate, citronellyl acetate, geranyl acetate, benzyl acetate, linalyl acetate, phenylethyl acetate, menthyl acetate, bornyl acetate, terpenyl acetate, cinnamyl acetate, anisyl acetate and myrcenyl acetate; and examples of propionates of an alcohol having from 5 to 10 carbon atoms include linalyl propionate, citronellyl propionate, geranyl propionate, benzyl propionate, terpenyl propionate and cinnamyl propionate.

Each of these highly volatile perfumes can be used alone in the bathing preparation of the present invention. Alternatively, a mixture thereof together with other perfumes may be used. The bathing preparation of the present invention preferably comprises 0.001 to 2% by weight, more preferably 0.005 to 0.6% by weight, of the highly volatile perfume(s) based on the total composition. Further, it is preferable that the total perfumes amount to 0.2 to 2% by weight of the bathing preparation.

In addition to the above-mentioned essential components, the bathing preparation of the present invention may further contain inorganic salts, for example, sulfates (e.g., sodium sulfate, magnesium sulfate and zinc sulfate) or chlorides (e.g., sodium chloride). These inorganic salts are preferably used in an amount of 0 to 20% by weight based on the total composition.

The bathing preparation of the present invention may furthermore contain various additives commonly used in bathing preparations to thereby enhance the effects. Examples of these additives include pigments, vitamins, active ingredients of hot springs, proteases, marine algae extracts, sodium alginate, lanolin, silicones, crude drugs or extracts thereof, and so on.

The bathing preparation of the present invention can provide carbon dioxide gas dissolved in bath water by appropriately selecting the composition ratio between the organic acid(s) and carbonate(s) in such a manner as to adjust the pH value of the bath water to 5 to 7. It is expected, in this case, that the dissolved carbon dioxide gas accelerates blood circulation.

It is preferable that the bathing preparation of the present invention is formulated into tablets since the dissolution of the carbon dioxide gas in bath water can be promoted thereby. However, it may be formulated into other forms such as a powder or granules. During the formulation step, conventional vehicles or lubricants may be used, if required.

Specific examples of the vehicles include sodium chloride, kaolin, carboxyvinyl polymer, powdered licorice, light silicic anhydride, synthetic aluminum silicate, magnesium silicate, calcium citrate, crystal cellulose, D-sorbitol, talc, precipitated calcium carbonate, dextrin, starch, tragacanth, lactose, sucrose, dextrose, D-mannitol, magnesium aluminum oxide metasilicate, aluminum monstearate, medical soap, calcium phosphate, calcium hydrogenphosphate, calcium sulfate, gum arabic, glycerin, syrupus simplex, aromatic powders, water, etc.

Specific examples of the lubricants include carnauba wax, light silicic anhydride, magnesium silicate, synthetic aluminum silicate, hardened oils, white Japan wax, titanium oxide, stearic acid, stearic acid salts (e.g., Al, K, Na, Ca, Mg), talc, corn starch, microcrystalline cellulose, Macrogol-4000, Macrogol-6000, isopropyl myristate, magnesium lauryl sulfate, calcium hydrogenphosphate, waxes, colloidal silicates (e.g., magnesium aluminum oxide silicate), etc.

As described above, the bathing preparation of the present invention comprising organic acids, including fumaric acid, at a definite level or above sustains specific highly volatile perfumes in bath water without vaporization, thus giving desirable bathing effects.

The present invention is now illustrated in greater detail by way of the following Examples wherein perfumes A to K shown in Table 1 were employed, but it should be understood that the present invention is not deemed to be limited thereto.

EP 0 339 276 B1

T A B L E   1

| Perfume | A | B | C | D | E | F | G | H | I | J | K |
|---|---|---|---|---|---|---|---|---|---|---|---|
| limonene | 60 | | | | 40 | | | 52 | 40 | 70 | 8 |
| pinene | 20 | | | | | | | | 20 | | |
| terpinolene | 20 | | | | 10 | | | 10 | | | 1 |
| geranyl formate | | 40 | | | 20 | | 30 | | 1 | 1 | |
| benzyl formate | | 30 | | | | 10 | | | | | |
| phenylethyl formate | | 30 | | | 30 | | | | | | |
| citronellyl acetate | | | 20 | | | | 20 | 2 | | 1 | 3 |
| geranyl acetate | | | 30 | | | | | | 5 | 1 | 3 |
| benzyl acetate | | | 10 | | | 10 | 10 | | 1 | | |
| phenylethyl acetate | | | 20 | | | 30 | | 4 | | | 1 |
| terpenyl acetate | | | 20 | | | | | | | | 3 |
| benzyl propionate | | | | 40 | | 50 | | | | 1 | 2 |
| isoamyl propionate | | | | 10 | | | 40 | | | 2 | |
| terpenyl propionate | | | | 50 | | | | | | | |
| lilial | | | | | | | | | | 10 | 15 |

T A B L E   1 (cont'd)

| Perfume | A | B | C | D | E | F | G | H | I | J | K |
|---|---|---|---|---|---|---|---|---|---|---|---|
| methyl dihydrodijasmonate | | | | | | | | | | 10 | 10 |
| cyclamen aldehyde | | | | | | | | 1 | | | 5 |
| isoamyl salicylate | | | | | | | | | 2 | | |
| methyl anthranilate | | | | | | | | 1 | | | 1 |
| methyl methylanthranilate | | | | | | | | | 1 | | 1 |
| methyl naphthyl ether | | | | | | | | 1 | | | |
| ethyl naphthyl ether | | | | | | | | | 1 | | 1 |
| tonalide | | | | | | | | | 4 | | |
| pentalide | | | | | | | | 4 | | | 10 |
| galaxolide | | | | | | | | 4 | 1 | 10 | 20 |
| ethyl vanillin | | | | | | | | 0.1 | | 1 | |
| anisic aldehyde | | | | | | | | | | | 1 |
| geraniol | | | | | | | | 5 | | | 8 |
| anethole | | | | | | | | | | 2 | 1 |
| phenylethyl alcohol | | | | | | | | 14.8 | 13 | | |

T A B L E     1 (cont'd)

| Perfume | A | B | C | D | E | F | G | H | I | J | K |
|---------|---|---|---|---|---|---|---|---|---|---|---|
| terpineol | | | | | | | | | 1 | | |
| damascone | | | | | | | | 0.1 | | 0.5 | |
| ionone | | | | | | | | 1 | | | 5 |
| arylamyl glycolate | | | | | | | | | | 0.5 | |
| cis-3-hexenyl salicylate | | | | | | | | | | | 1 |

EXAMPLE 1

Composition :

| | |
|---|---|
| Sodium Hydrogencarbonate | 43.8 (wt%) |
| Sodium Carbonate | 15 (wt%) |
| Organic Acids (Table 2) | 40(wt%) |
| Dextrin | 0.8 (wt%) |
| Perfume A | 0.4 (wt%) |

A bathing preparation of the above composition was mixed and tableted. 50 g of the bathing preparation was introduced into a bath tub (910 × 710 cm) filled with 150 liters of water at 40°C and the aroma was evaluated over the course of time by experienced panelists according to the following criterion:

○: full aroma;

Ⓐ: moderate aroma;

Δ: slight aroma; and

×: no aroma.

The evaluations ○ and Ⓐ were satisfactory as a bathing preparation.

The results are shown in Table 2 below.

EP 0 339 276 B1

T A B L E   2

| | No. 1 | No. 2 | No. 3 | No. 4 | No. 5 | No. 6 | No. 7 | No. 8 |
|---|---|---|---|---|---|---|---|---|
| **Organic Acid (wt%)** | | | | | | | | |
| succinic acid | 100 | 90 | 80 | 60 | 40 | 20 | 0 | 0 |
| fumaric acid | 0 | 10 | 20 | 40 | 60 | 80 | 100 | 20 |
| tartaric acid | 0 | 0 | 0 | 0 | 0 | 0 | 0 | 80 |
| **Aroma** | | | | | | | | |
| at disintegration | △ | △ | ○ | ○ | ○ | ○ | ○ | ○ |
| after 5 minutes | × | △ | ⊚ | ○ | ○ | ○ | ○ | ⊚ |
| after 15 minutes | × | × | ⊚ | ○ | ○ | ○ | ○ | ⊚ |
| after 60 minutes | × | × | ⊚ | ⊚ | ○ | ○ | ○ | ⊚ |

From the results shown in Table 2, it can be seen that the bathing preparation containing 20% by weight or more of fumaric acid as an organic acid sustains the aroma thereof for a prolonged period of time.

## EXAMPLE 2

Composition :

| | |
|---|---|
| Sodium Hydrogencarbonate | 43.8 (wt%) |
| Sodium Carbonate | 15 (wt%) |
| Organic Acids (Table 3) | 40 (wt%) |
| Dextrin | 0.8 (wt%) |
| Perfume (Table 3) | 0.4 (wt%) |

A bathing preparation of the above composition was mixed, tableted and introduced into a bath tub in the same manner as described in Example 1. 60 minutes after the introduction, the aroma of the bath water was evaluated by experienced panelists similar to Example 1.

The results are shown in Table 3 below.

### TABLE 3

| Perfume | No.9 B | No.10 B | No.11 C | No.12 D | No.13 E | No.14 F | No.15 G |
|---|---|---|---|---|---|---|---|
| **Organic Acid (wt%)** | | | | | | | |
| succinic acid | 90 | 50 | 50 | 50 | 50 | 50 | 50 |
| fumaric acid | 10 | 50 | 50 | 50 | 50 | 50 | 50 |
| Aroma after 60 min. | × | ○ | ⊘ | ○ | ⊘ | ○ | ○ |

From the results shown in Table 3, it can be seen that the bathing preparation containing 20% by weight or more (for example, 50% by weight) of fumaric acid as an organic acid sustains the aroma thereof for a prolonged period of time in spite of the kind of perfume.

## EXAMPLE 3

A bathing preparation of the same composition as described in Example 2 but using the organic acids and perfumes shown in Table 4 was mixed, tableted and introduced into a bath tub similar to Example 1. 60 minutes after the introduction, the aroma of the bath water was organoleptically evaluated by experienced panelists in the same manner as the one described in Example 1.

The results are shown in Table 4 below.

## TABLE 4

| Perfume | No. 16 H | No. 17 H | No. 18 I | No. 19 J | No. 20 K |
|---|---|---|---|---|---|
| **Organic Acid (wt%)** | | | | | |
| succinic acid | 100 | 40 | 40 | 40 | 40 |
| fumaric acid | 0 | 60 | 60 | 60*1 | 60*2 |
| Aroma after 60 min. | × | ◬ | ○ | ○ | ◬ |

Note: 1) 95.20 wt% of fumaric acid was surface-treated by a heat-molten process with 4.75 wt% of polyethylene glycol (average molecular weight: 6,000) and 0.05 wt% of sucrose fatty acid ester (HLB 15).

2) 88 wt% of fumaric acid was surface-treated by a spray drying process with 12 wt% of sodium polyacrylate (average molecular weight: 8,000).

From the results shown in Table 4, it can be seen that the bathing preparation containing 20% by weight or more (for example, 60% by weight) of fumaric acid as an organic acid sustains the aroma thereof for a prolonged period of time in spite of the kind of perfume.

While the invention has been described in detail and with reference to specific embodiments thereof, it will be apparent to one skilled in the art that various changes and modifications can be made therein without departing from the spirit and scope thereof.

The registered trade marks as used in the above aknowledged as such.

## Claims

1. A bathing preparation comprising:
(A) at least one organic acid, wherein fumaric acid must be present in an amount of 20% by weight or more of the total of said organic acid,
(B) at least one carbonate, and
(C) at least one perfume selected from the group consisting of terpene hydrocarbons having 10 carbon atoms and formates, acetates and propionates of an alcohol having from 5 to 10 carbon atoms.

2. The bathing preparation as claimed in Claim 1, wherein said carbonate is selected from the group consisting of sodium hydrogencarbonate, sodium carbonate, potassium hydrogencarbonate, potassium carbonate, calcium carbonate, magnesium carbonate and sodium sesquicarbonate and mixtures thereof.

3. The bathing preparation as claimed in Claim 1, wherein said carbonate is employed in an amount of from 5 to 80% by weight based upon the total composition.

4. The bathing preparation as claimed in Claim 3, wherein said carbonate is employed in an amount of from 10 to 50% by weight based upon the total composition.

5. The bathing preparation as claimed in Claim 1, wherein said organic acid is a mixture of fumaric acid and at least one organic acid selected from the group consisting of citric acid, succinic acid, malic acid and tartaric acid.

6. The bathing preparation as claimed in Claim 1, wherein the weight of organic acid to said carbonate is from 0,1:1 to 3:1.

7. The bathing preparation as claimed in Claim 6, wherein the weight ratio of organic acid to said carbonate is from 0,3:1 to 1,5:1.

8. The bathing preparation as claimed in Claim 1, wherein said fumaric acid is surface-treated with a water-soluble polymer, a hydrophilic nonionic surfactant or mixture thereof.

9. The bathing preparation as claimed in Claim 1, wherein said terpene hydrocarbons having 10 carbon atoms is selected from the group consisting of α-pinene, β-pinene, camphene, limonene, terpinolene, myrcene and p-cymene; wherein said formates of an alcohol is selected from the group consisting of geranyl formate, benzyl formate and phenylethyl formate; wherein said acetate of an alcohol is selected from the group consisting of isoamyl acetate, citronellyl acetate, geranyl acetate, benzyl acetate, linalyl acetate, phenylethyl acetate, menthyl acetate, bornyl acetate, terpenyl acetate, cinnamyl acetate, anisyl acetate and myrcenyl acetate; and wherein said propionate of an alcohol is selected from the group consisting of linalyl propionate, citronellyl propionate, geranyl propionate, benzyl propionate, terpenyl propionate and cinnamyl propionate.

10. The bathing preparation as claimed in Claim 1, wherein said perfume is employed in an amount of from 0.001 to 2% by weight based on the total composition.

11. The bathing preparation as claimed in Claim 10, wherein said perfume is employed in an amount of from 0.005 to 0.6% by weight based on the total composition.


**Patentansprüche**

1. Badezubereitung, umfassend:
(A) wenigstens eine organische Säure, wobei Fumarsäure in einer Menge von 20 Gew.-% oder mehr der gesamten organischen Säure vorhanden sein muß,
(B) wenigstens ein Carbonat und
(C) wenigstens ein Parfüm, ausgewählt aus der Gruppe bestehend aus Terpenkohlenwasserstoffen mit 10 Kohlenstoffatomen und Formiaten, Acetaten und Propionaten eines Alkohols mit 5 bis 10 Kohlenstoffatomen.

2. Badezubereitung gemäß Anspruch 1, in welcher das Carbonat ausgewählt ist aus der Gruppe bestehend aus Natriumhydrogencarbonat, Natriumcarbonat, Kaliumhydrogencarbonat, Kaliumcarbonat, Kalziumcarbonat, Magnesiumcarbonat und Natriumsesquicarbonat und Mischungen davon.

3. Badezubereitung gemäß Anspruch 1, in welcher das Carbonat in einer Menge von 5 bis 80 Gew-%, bezogen auf das die gesamte Zusammensetzung, verwendet wird.

4. Badezubereitung gemäß Anspruch 3, in welcher das Carbonat in einer Menge von 10 bis 50 Gew.-%, bezogen auf die gesamte Zusammensetzung, verwendet wird.

5. Badezubereitung gemäß Anspruch 1, in welcher die organische Säure eine Mischung aus Fumarsäure und wenigsten einer Organischen Säure ausgewahlt aus der Gruppe bestehend aus Zitronensäure Bernsteinsäure Apfelsäure und Weinsäure ist.

6. Badezubereitung gemäß Anspruch 1, in welcher das Gewichtsverhältnis der organischen Säure zu dem Carbonat 0,1 : 1 bis 3 : 1 beträgt.

7. Badezubereitung gemäß Anspruch 6, in welcher das Gewichtsverhältnis der organischen Säure zu dem Carbonat 0,3 : 1 bis 1,5 : 1 beträgt.

8. Badezubereitung gemäß Anspruch 1, in welcher die Fumarsäure mit einem wasserlöslichen Polymer, einem hydrophilen nichtionischen oberflächenaktiven Mittel oder einer Mischung daraus oberflächenbehandelt ist.

9. Badezubereitung gemäß Anspruch 1, in welcher die Terpenkohlenwasserstoffe mit 10 Kohlenstoffatomen ausgewählt sind aus der Gruppe bestehend aus alpha-Pinen, beta-Pinen, Camphen, Limonen, Terpinolen, Myrcen und p-Cymol; worin das Formiat eines Alkohols ausgewählt ist aus der Gruppe bestehend aus Geranylformiat, Benzylformiat und Phenylethylformiat; worin das Acetet eines Alkohols ausgewählt ist aus der Gruppe bestehend aus Isoamylacetat, Citronellylacetat, Geranylacetat, Benzylacetat, Linalylacetat, Phenylethylacetat, Menthylacetat, Boinylacetat, Terpenylacetet, Cinnamylacetat, Anisylacetat und Myrcenylacetat, und worin das Propionat eines Alkohols ausgewählt ist aus der Gruppe bestehend aus Linalylpropionat, Citronellylpropionat, Geranylpropionat, Benzylpropionat, Terpenylpropionat und Cinnamylpropionat.

10. Badezubereitung gemäß Anspruch 1, in welcher das Parfüm in einer Menge von 0,001 bis 2 Gew.-%, bezogen auf die gesamte Zusammensetzung, verwendet wird.

11. Badezubereiten gemäß Anspruch 10, in welcher das Parfüm in einer Menge von 0,005 bis 0,6 Gew.-%,

bezogen auf die gesamte Zusammensetzung, verwendet wird.

**Revendications**

1. Produit pour le bain comprenant:
(A) au moins un acide organique dans lequel l'acide fumarique doit être présent en une quantité supérieure ou égale à 20 % en poids du total dudit acide organique,
(B) au moins un carbonate et
(C) au moins un parfum sélectionné dans le groupe constitué par des hydrocarbures terpéniques ayant 10 atomes de carbone et des formiates, des acétates et des propionates d'un alcool ayant de 5 à 10 atomes de carbone.

2. Produit pour le bain selon la revendication 1, dans lequel ledit carbonate est sélectionné dans le groupe constitué par l'hydrogénocarbonate de sodium, le carbonate de sodium, l'hydrogénocarbonate de potassium, le carbonate de potassium, le carbonate de calcium, le carbonate de magnésium et le sesquicarbonate de sodium et des mélanges de ceux-ci.

3. Produit pour le bain selon la revendication 1, dans lequel ledit carbonate est employé en une quantité comprise entre 5 et 80% en poids par rapport à la composition totale.

4. Produit pour le bain selon la revendication 3, dans lequel ledit carbonate est employé en une quantité comprise entre 10 et 50% en poids par rapport à la composition totale.

5. Produit pour le bain selon la revendication 1, dans lequel ledit acide organique est un mélange d'acide fumarique et d'au moins un acide organique sélectionnné dans le groupe constitué par l'acide citrique, l'acide succinique, l'acide malique et l'acide tartrique.

6. Produit pour bain selon la revendication 1, dans lequel le rapport pondéral de l'acide organique audit carbonate est compris entre 0,1:1 et 3:1.

7. Produit pour le bain selon la revendication 6, dans lequel le rapport pondéral de l'acide organique audit carbonate est compris entre 0,3:1 et 1,5:1.

8. Produit pour le bain selon la revendication 1, dans lequel ledit acide fumarique est soumis à un traitement superficiel à l'aide d'un polymère hydrosoluble, d'un surfactant non ionique hydrophile ou d'un mélange de ceux-ci.

9. Produit pour le bain selon la revendication 1, dans lequel ledit hydrocarbure terpénique possédant 10 atomes de carbone est sélectionné dans le groupe constitué par l'α-pinène, le β-pinène, le camphène, le terpinolène, le myrcène et le p-cymène ; dans lequel ledit formiate d'un alcool est sélectionné dans le groupe constitué par le formiate de géranyle, le formiate de benzyl et le formiate de phényléthyle ; dans lequel ledit acétate d'un alcool est sélectionné dans le groupe constitué par l'acétate d'isoamyle, l'acétate de citronellyle, l'acétate de géranyle, l'acétate de benzyle, l'acétate de linalyle, l'acétate de phényléthyle, l'acétate de menthyle, l'acétate de bornyle, l'acétate de terpényle, l'acétate de cinnamyle, l'acétate d'anisyle et l'acétate de myrcényle ; et dans lequel ledit propionate d'un alcool est sélectionné dans le groupe constitué par le propionate de linalyle, le propionate de citronellyle, le propionate de géranyle, le propionate de benzyle, le propionate de terphényle et le propionate de cinnamyle.

10. Produit pour le bain selon la revendication 1, dans lequel ledit parfum est employé en une quantité comprise entre 0,001 et 2% en poids par rapport à la composition totale.

11. Produit pour le bain selon la revendication 10, dans lequel ledit parfum est employé en une quantité comprise entre 0,005 et 0,6 % en poids par rapport à la composition totale.